# EUROPEAN PATENT APPLICATION

(11) **EP 4 198 998 A1**
(43) Date of publication of application: **21.06.2023**
(21) Application number: 21215664.0
(22) Date of filing: 17.12.2021
(51) Int. Cl.: G16H 30/40, G16H 40/63, G16H 50/50

(54) **AIDING AN ANALYSIS OF AN ANATOMICAL ELEMENT OF A SUBJECT**

(71) Applicant: Koninklijke Philips N.V., 5656 AG Eindhoven (NL)
(72) Inventor: WILDEBOER, Rogier Rudolf, Eindhoven (NL); RUETTEN, Walter, Eindhoven (NL); KOLEN, Alexander, Eindhoven (NL); CLUITMANS, Matthijs Joseph Maria, Eindhoven (NL); HENDRIKS, Bernardus Hendrikus Wilhelmus, Eindhoven (NL); KAHLMAN, Josephus Arnoldus Johannes Maria, Eindhoven (NL)
(74) Representative: Philips Intellectual Property & Standards

(57) **Abstract**

A mechanism for identifying regions of an anatomical element that are insufficiently represented by the anatomical model and/or are sites of a local structural or electrical abnormality. An anatomical model and a measured electrical activity model are obtained. The anatomical model is processed to produce a predicted electrical activity model. The measured and predicted electrical activity models are compared to identify any regions of mismatch, which represent regions underrepresented by the anatomical model and/or sites of abnormalities.

## Description

### FIELD OF THE INVENTION

The present invention relates to the medical field of subject analysis, and in particular to the analysis of anatomical elements.

### BACKGROUND OF THE INVENTION

There is an increasing interest in the (computer) modelling of anatomical elements or structures of a subject, to provide a visual representation of the anatomical element. This aids analysis of the anatomical element, e.g. during the course of a medical procedure or diagnosis.

One area of particular interest is in electro-anatomical mapping of an anatomical element (such as the heart or parts thereof), in which procedures one or more catheters are inserted into an anatomical cavity of the anatomical element and an electro-anatomical mapping system is used to localize the catheters and possibly generate an anatomical model representing the structure of the anatomical element or part thereof. Examples of such systems include the CARTO^{®} system of Biosense Webster^{®}, the EnSite^{®} system of Abbott^{®} and the KODEX-EPD^{®} system of Philips^{®}, but others exist. These exploit magnetic based 3D localization and/or dielectrics based 3D localization of the tip of a catheter to allow radiation-free navigation in an anatomical cavity and enable radiation free modelling of the inside surface of the anatomical cavity to represent the structure of the anatomical element investigated.

Dielectrics based 3D localization techniques, such as those employed by the KODEX-EPD system, are based on electrical fields applied to the body using three pairs of external body electrode-patches that represent the spatial x-, y-, and z-dimension. During a procedure a catheter is entered into and moved about in an anatomical cavity of an anatomical element to which the electric fields are applied while measuring the field using electrodes on the tip of the catheter. The measured data are subsequently transformed to positions of the electrodes in a physical space using an appropriate transformation function. This enables localization of the electrodes and therewith catheter tip in space or a coordinate system. At the same time, the transformed measurements are used to reconstruct an anatomical model of the anatomical element. The catheter position can then be determined with respect to the anatomical model, allowing accurate navigation towards and around anatomical features.

There is a desire to improve accuracy of the anatomical modelling.

### SUMMARY OF THE INVENTION

The invention as defined by the claims at least partly fulfil this desire.

According to examples in accordance with an aspect of the current disclosure, there is provided a processing system for aiding an analysis of an anatomical element of a subject. The processing system comprises: a processing unit and an input interface and an optional output interface where the input and output interfaces are communicatively coupled to the processing unit.

The input interface is configured to receive model data representative of an anatomical model of at least a part of (or all of) the anatomical element. Model data is based on measurements recorded with one or more position sensors on a catheter while moving the catheter around in a cavity of the anatomical element. As such the model data may be processed into, represents, or comprises the anatomical model. The input interface is also configured to receive electrical activity data representative of a measured electrical activity of the anatomical element. Such electrical activity may for example be electrical activity of a heart or parts thereof. The electrical activity data may represent or comprise an electrical activity model.

The processing unit is configured to process the model data and, using the anatomical model and an electrophysical simulation technique, to generate predicted electrical activity data for the at least part of the anatomical element.

The optional output interface is configured to provide output data or output information representative of a comparison of some, or all, of the predicted electrical activity data and some, or all, of the corresponding measured electrical activity data. Corresponding means that the predicted and measure data pertain to a same portion of the anatomical element and/or anatomical model. The provision of output data may also be responsive to the comparison. A comparison can be in the form of a simultaneous output of the predicted and measured data for the one or more regions. Alternatively or additionally the output may have other forms as will be defined and described herein.

The comparison allows a user to identify one or more regions of the anatomical element that are insufficiently represented by the anatomical model. Alternatively or additionally such comparison allows identification of sites of a local structural or electrical abnormality in the anatomical element.

The present disclosure provides an approach for identifying areas of an anatomical element that are either abnormal or have been insufficiently mapped during a mapping procedure. In particular, an anatomical model (e.g. an anatomical map or mesh) representing the anatomical element or part thereof is processed using an electrophysical simulation technique to generate measures of electrical activity (for the anatomical element or the part thereof). These measures of electrical activity may form a predicted electrical activity model, which represents predicted electrical activity at different locations of the anatomical element and/or of the anatomical model. The predicted electrical activity model may then be compared to the measured electrical activity model. The comparison may identify true or measured values of electrical activity in the anatomical element. The comparison may be used to identify one or more regions of mismatch, i.e. regions of the anatomical element or anatomical model where the predicted and measured electrical activity disagree with one another.

The proposed approach advantageously facilitates identification of areas in the form of the mismatch regions that would benefit from further mapping, i.e. areas that are not correctly mapped or show some form of abnormality. This provides useful feedback for clinicians, so that they are able to (for instance) remap any identified region and/or take identified inaccuracies into account when diagnosing or assessing the subject based on the anatomical model and/or measured electrical activity model. Thus, proposed approaches provide useful clinical information for aiding a clinician in making a clinical decision (e.g. whether to perform additional mapping or identifying locations of potential abnormalities) about the subject.

The model data and therewith anatomical model may be generated using any suitable approach using monitored locations of a catheter, e.g. any suitable mapping approach. Such approaches are well established in the art, and may employ monitoring the response of one or more position sensors on a catheter to magnetic and/or electric fields induced in the anatomical cavity.

If present, the output interface may be configured to control a user interface to provide a visual representation of the comparison, e.g. provide a visual representation of at least some of the predicted electrical activity data and at least some of the corresponding measured electrical activity data. Such comparison may comprise an indication of identified regions of mismatch. This may be in the form of a visual representation of the anatomical element (e.g. generated based on the anatomical model) with visual indicators identifying any regions of mismatch.

In the context of the present disclosure, an anatomical element may comprise an entire anatomical feature (e.g. an organ) or part of the anatomical feature. Thus, the anatomical model may effectively be a partial anatomical model of a larger anatomical feature such as an organ, i.e. the anatomical model may only model/represent part (i.e. not all) of the organ, or a complete anatomical model of the entire anatomical feature.

It is emphasized that the proposed approach can be performed entirely separately of the investigation of the anatomical element using the catheter to generate model data and/or measured electrical activity data. In particular, the proposed approach could be performed using previously collected data, computer-simulated data, dummy data, data from literature and/or data collected from cadavers and stored in a memory of any kind. There is no direct link between the collection or generation of the data and the process proposed by the present disclosure.

In some examples, the model data may comprise the anatomical model. In such case the model data may comprise positional coordinates in a physical coordinate space of an anatomical model such as e.g. a mesh model. Alternatively, or additionally, the processing unit is configured to process the model data to generate the anatomical model. In such case the model data may comprise measurement data representative of a magnetic and/or electric field applied to an anatomical element which has been probed over time with the appropriate field sensors as positional sensors located on a catheter while exploring a cavity of the anatomical element. Such data can be processed to be transformed to positional coordinates of the sensors and from such positional coordinates the anatomical model or part thereof may be constructed. The model data can also comprise the positional coordinates perse such that the processing may comprise construction of the anatomical model without the transformation of any measured model data. A combination of any of the above to generate an anatomical model may also be used.

The model data may comprise magnetic field data, electric field data or both. Typically, such data are AC field data. Any parameters representative of such fields and dependent on the catheter locations in a space may be used. For example, magnetic field data may have amplitudes distinguishable along three crossing directions. Electric field data may for example comprise voltage data, current data or derivatives thereof such as impedance, gradients etc distinguishable in at least three directions.

In some embodiments the processing unit is configured to compare some of the measured electrical activity data with some of the predicted electrical activity data. In some embodiments the processing unit is configured to identify one or more any regions of mismatch between the predicted electrical activity data (model) and the measured electrical activity data (model). In some examples, a region of mismatch is a region in which a difference between a value of the predicted electrical activity data (model) and the measured electrical activity data (model) differs by more than a threshold amount. The threshold amount may be fixed within the processing unit. Alternatively the threshold may be user definable and in such case the processing system may be configured to receive a threshold value via a user input interface upon input by a user via an input device operably connected to the input interface. Such value may be stored in a memory for use by the processing unit.

This embodiment provides a simple mechanism for assessing whether a region is a mismatch or whether a difference is due to noise, natural variation and/or expected errors or inaccuracies of the electrophysical stimulation technique. A robustness of the step for identifying appropriate regions is therefore improved.

In some examples, the electrophysical simulation technique comprises a finite-element simulation that models electro-activation of the anatomical element. Model input parameters such as wall thickness, fiber orientation, conduction velocity, tissue characteristics etc. may be taken from literature values, generic databases, patient-specific preprocedural images, directly from intraprocedurally recorded measures, or from data from previous procedures. A finite-element simulation provides a good working example of how to model electrical activity in an anatomical model, which employs well-established techniques to provide accurate and reliable prediction of electrical activity.

In at least one embodiment, the processing unit is further configured to generate an uncertainty map identifying the position of the regions of mismatch with respect to the anatomical model. An uncertainty map would provide a clinician with useful information for easily and readily identifying regions that would benefit from further investigation and/or analysis. In particular, an uncertainty map may identify areas worthy of further mapping (e.g. to update and/or correct the anatomical model).

Optionally, the electrophysical simulation technique comprises a simulation of electrical activity in an anatomical structure defined by the anatomical model.

In some embodiments, the electrophysical simulation technique comprises using values contained in the measured electrical activity model as boundary conditions for the simulation of electrical activity. This approach improves the accuracy and reliability of the electrophysical simulation technique. In particular, some of the values of the measured electrical activity model may be used as boundary conditions, with other values being used to assess or identify regions of mismatch (i.e. regions of mismatch).

In at least one example, the input interface is further configured to receive preoperative imaging data representative of a pre-operative anatomical model, wherein the pre-operative anatomical model is/was generated from an imaging procedure; and the processing unit is further configured to: spatially register the pre-operative anatomical model to the anatomical model; and compare and/or identify any regions of the anatomical element represented in the pre-operative anatomical model but not represented in the anatomical model.

This approach provides a further method of identifying regions of the anatomical model that are insufficiently mapped or represented by the anatomical model. This provides additional information for a clinician in assessing the anatomical model and/or deciding whether and where to perform further mapping of the anatomical model. The imaging procedure may be a non-invasive imaging procedure, to reduce a number of intrusive actions performed on the subject.

Such pre-operative imaging data may be generated using one or more of : X-Ray, CT, MRI and Ultrasound imaging of the anatomical element or parts thereof, preferably in 3D. Such data may be used to construct anatomical models as known in the art.

The processing unit may be configured to spatially register the pre-operative anatomical model to the anatomical model by: identifying the same one or more landmark features in the pre-operative anatomical model and the anatomical model; and based on the identified landmark features, spatially registering the pre-operative anatomical to the anatomical model.

Optionally, the input interface is further configured to receive a pre-operative anatomical model, wherein the pre-operative anatomical model is/was generated from a non-invasive imaging procedure; and the processing unit is further configured to process the pre-operative anatomical model using an electrophysical simulation technique to generate a second predicted electrical activity model.

In this embodiment, the processing unit may be further configured to: identify any regions of mismatch between the second predicted electrical activity model and the measured electrical activity model, to thereby identify any regions of the anatomical element that are probable sites of a local structural or electrical abnormality in the anatomical element; and/or identify any regions of mismatch between the second predicted electrical activity model and the predicted electrical activity model, to thereby identify any regions of the anatomical element that are probable insufficiently represented by the anatomical model.

Optionally, the input interface is further configured to receive electrocardiogram data identifying any occurrences of arrhythmic cardiac activity; and the processing unit is configured to discount, from the measured electrical activity data (model), any measures of electrical activity measured during any identified occurrence of arrhythmic cardiac activity.

An arrhythmic cardiac event will cause unusual or unexpected electrical activity in an anatomical element of a subject. By discounting such electrical activity (e.g. by monitoring electrocardiogram data), a chance of a region of mismatch representing a true area requiring further mapping and/or a (physical) abnormality is increased - rather than simply representing an area that was measured when a cardiac event occurred. This embodiment thereby improves the accuracy and reliability of identifying regions of the anatomical element that are either insufficiently represented by the anatomical model and/or sites of a local abnormality in the anatomical element.

Optionally, the anatomical element is a heart of the subject or a part of such heart. Typical parts are left or right atrium and or left or right ventricles and or one or more pulmonary veins. Embodiments are particularly advantageous for such elements, because electrical activity of the heart is a well-established area with significant interest in accurate modelling and identification of electrical activity values. Thus, existing reliable and robust approaches for modeling electrical activity of the heart can be employed to advantage in identifying regions that would benefit from further investigation and/or mapping.

The measures of electrical activity may be measures of activation voltages and/or local activation times. Other suitable measures of electrical activity will be readily apparent to the skilled person, e.g. a measure of voltage, impedance, or electrical current.

Optionally, the electrophysical simulation technique comprises a process of simulating local point-to-point or local surface area propagation of an electrical pulse with respect to a heart structure represented by the anatomical model to generate predictive relative activation times between different areas of the heart structure represented by the anatomical model; and the measured electrical activity model comprises measures of relative activation times between the different areas of the heart structure represented by the anatomical model.

Of course, the anatomical element may be another part of the subject, such as the lungs, liver, kidney and so on.

The processing unit may be configured to generate, for each identified region of mismatch, an indicator of the severity of the mismatch based on the difference between the predicted electrical activity, provided by the predicted electrical activity model, and the measured electrical activity, provided by the measured electrical activity model, at the region of mismatch. The output data may be representative of such indicator.

Measures of severity provide useful information for a clinician to assess whether additional mapping and/or investigation of a particular region is worthwhile, e.g. taking into account the risk/difficulty of additional mapping (which requires intervention) and likely benefit to improving the accuracy of the anatomical model. Thus, any measures of severity would provide useful clinical information for making a clinical decision about a subject.

The current disclosure also provides a medical treatment system comprising the processing system of any of the previous claims. Such treatment system may comprise or consist of an electromagnetic or dielectric imaging system and/or an electro-anatomical mapping system. Such system may also include other components such as one or more signal generator measurers to control various parts of the treatment system. Such parts may include one or more catheters and the position sensors thereon as well as one or more field generators used to create the appropriate electromagnetic or electric fields. Such systems may also include ablation subsystems for treatment of a condition by ablating tissue of an anatomical element. Such treatment systems may also include user interfaces for conveying information to a user and user interfaces for controlling the system.

There is also proposed a computer-implemented method for aiding an analysis of an anatomical element of a subject.

The computer-implemented method comprises: receiving, at an input interface: model data representative of an anatomical model of at least a part of the anatomical element, and measured electrical activity data of the at least part of the anatomical element.

The computer-implemented method further comprises processing, using a processing unit, the anatomical model using an electrophysical simulation technique to generate predicted electrical activity data and, optionally, providing at an output interface output data representative of a comparison of the at least some of the predicted electrical activity data with at least some of the corresponding measured electrical activity data.

The method may include the processing unit performing comparing (530) at least some of the predicted electrical activity data with the measured electrical activity data. Alternatively or additionally, the method may include the processing unit performing identifying one or more regions of mismatch between the at least some of the predicted electrical activity data and the at least some of the measured electrical activity data. The method may include the processing unit to generate an indication of the identified one or more regions and/or, based on such one or more regions, an indication that the one or more regions of the anatomical element are either insufficiently represented by the anatomical model and/or sites of a local structural or electrical abnormality in the anatomical element. Any of these indications may be comprised in the output data. The output data may be responsive to the identified regions of mismatch.

The computer-implemented method may be modified to perform steps carried out by any herein described processing system and/or unit, and vice versa.

There is also proposed a computer program product comprising computer program code means which, when executed on a processing unit of a processing system, cause the processing system to perform all of the steps of any herein described method.

Any of the data described and processed herein may be embodied or comprised in appropriate signals such as electrical and/or optical signals that can be handled by circuit devices part of processing units and processing systems as described herein.

These and other aspects of the disclosure will be apparent from and elucidated with reference to the embodiment(s) described hereinafter.

### BRIEF DESCRIPTION OF THE DRAWINGS

For a better understanding of the disclosure, and to show more clearly how it may be carried into effect, reference will now be made, by way of example only, to the accompanying schematic drawings, in which:
Figure 1 illustrates a system for generating models for use in embodiments;
Figure 2 conceptually illustrates an approach for generating an anatomical model from a cloud of points;
Figure 3 illustrates a system containing a processing system according to an embodiment;
Figure 4 illustrates a variation to the processing system;
Figure 5 illustrates a method according to an embodiment; and
Figure 6 illustrates a processing system according to an embodiment; and
Figure 7 illustrates a system.

### DETAILED DESCRIPTION OF THE EMBODIMENTS

The disclosure will be described with reference to the Figures.

The detailed description and specific examples, while indicating exemplary embodiments of the apparatus, systems and methods, are intended for purposes of illustration only and are not intended to limit the scope of the disclosure. These and other features, aspects, and advantages of the apparatus, systems and methods of the present disclosure will become better understood from the following description, appended claims, and accompanying drawings. The Figures are merely schematic and are not drawn to scale. The same reference numerals are used throughout the Figures to indicate the same or similar parts.

The disclosure provides a mechanism for identifying regions of an anatomical element that are insufficiently represented by the anatomical model and/or sites of a local structural or electrical abnormality. An anatomical model and a measured electrical activity model are obtained. The anatomical model is processed to produce a predicted electrical activity model. The measured and predicted electrical activity models are compared to identify any regions of mismatch, which represent regions underrepresented by the anatomical model and/or sites of abnormalities.

The present disclosure is based on the realization that an inaccurate anatomical model will produce a mismatch between predicted electrical activity and actual (measured) electrical activity. Simultaneously, it is appreciated that areas of abnormality will also produce such a mismatch, as the prediction may not accurately represent the true nature of the electrical activity (as such predictions will assume normality in that region). This produces useful information for a clinician to decide where further investigation is merited, e.g. to improve a knowledge of the region of mismatch. Approaches thereby provide useful information for assessing and/or investigating a subject.

Embodiments may be employed in any suitable clinical environment in which anatomical models and electrical activity of an anatomical element can be determined, e.g. during catheter intervention into the heart.

Approaches adopted by the envisaged system make use of an anatomical model and a measured electrical activity model of an anatomical element. The anatomical element may form part of a larger anatomical structure, e.g. which has yet to be fully investigated. Approaches for generating such models using a catheter that can be positioned within an anatomical cavity of the anatomical element are hereafter described.

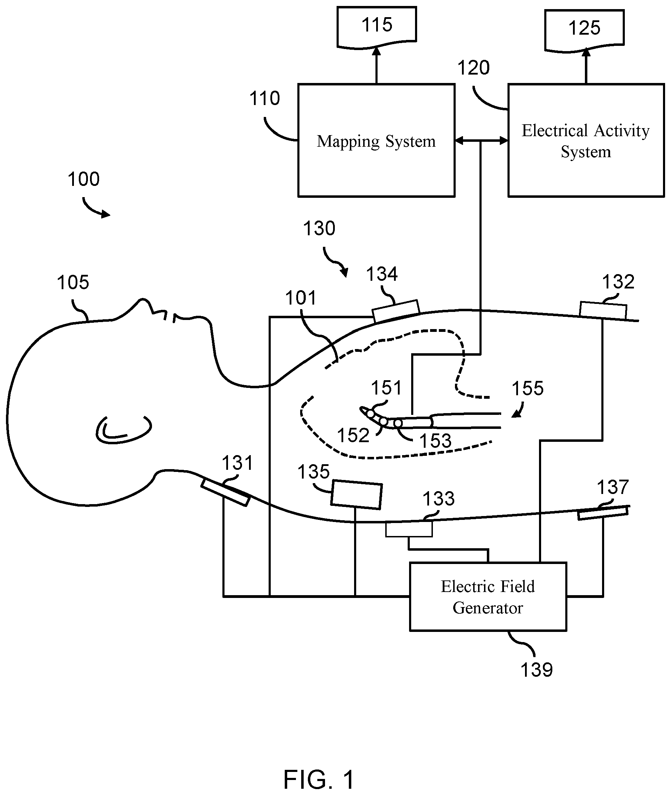

Figure 1 conceptually illustrates a system 100 for analysis of an anatomical element 101 of a subject 105. The anatomical element may, for instance, be a heart or vascular entity of the subject or a part thereof, although any other suitable anatomical element with an anatomical cavity (e.g. liver, kidney, lungs etc.) could be used. In this case the anatomical element 101 represents a left or right atrium or left or right ventricle of a heart.

The system 100 comprises a mapping system 110 and an electric field generator 139 together configured to generate an anatomical model 115 of the anatomical element 101. Thus in this case the mapping system is a dielectric imaging system, but this is not necessary per se. The anatomical model is/was generated by monitoring the location of position sensors (electrodes) on a distal tip of a catheter 155 as the distal tip moves within an anatomical cavity of the anatomical element while the electric field generator applies an electric field to the element 101 using the external body electrodes 131, 132, 133, 134, 135, 136 (not shown for clarity), 137.

Generally, construction of an anatomical model is performed by first generating a cloud of points within a defined space, each point representing a position or location of the catheter (and/or a sensor electrode on the catheter) within a defined space. This cloud of points is subsequently processed to construct an anatomical model.

One approach, not shown in Figure 1, for monitoring the location of a catheter 155 as the catheter moves within the anatomical cavity is to use a magnetic sensor(s) in the distal tip of a catheter that respond to crossing magnetic fields induced within the subject. Different magnetic responses of the magnetic sensor represents a different location of the magnetic sensor in a defined space. In this way, as the catheter is moved within the anatomical cavity, a collection or cloud of points can be constructed from the magnetic responses of the magnetic sensor. Approaches for monitoring the location using a magnetic sensor are established in the art, for instance as described by, inter alia, WO 2017/201288 A1, US 2021/052191 A1 and WO 2012/150567 A1. Any of these approaches, or others established in the art, could be employed for use in the present disclosure.

Another approach as represented with Figure 1 for monitoring the location of a catheter 155 as the catheter moves within the anatomical cavity is to monitor the electrical response of one or more electrodes of the catheter ("internal electrodes") to crossing electric fields induced within the subject.

An electric field generating arrangement 130 may be configured to generate these crossing electrical fields using a set of external electrodes 131, 132, 133, 134, 135, 137, which are positioned externally with respect to the subject 105. This is performed by an electric field generator 139 appropriate controlling characteristics (e.g. voltage and/or current) of the electric signals provided to each external electrode. Electrode 136 is not shown for clarity but would be located opposite to electrode 135 under the left arm pit of the subject.

The crossing electric fields may be controlled to have a different frequency with respect to one another, which effectively facilitates triangulation of the electrode within an electric field space.

The set 130 of external electrodes may comprise a plurality of electrode pairs angled with respect to one another (e.g. orthogonal to one another), so that any electric fields generated by the electrode pairs are angled with respect to one another. These electrode pairs may comprise a first electrode pair (formed of a first 131 and second 132 external electrode), a second electrode pair (formed of a third 133 and fourth 134 external electrode) and a third electrode pair (formed of a fifth 135 and sixth (not visible) external electrode). One of more of these electrode pairs may be omitted. The set of external electrodes may also comprise a reference electrode 137.

The electric field generator 139 may be configured to control the electric fields to operate in the frequency range of 10-1000 kHz, e.g. 10-500 kHz, e.g. 10-300kHz, e.g. 30-500kHz. These frequency ranges are particularly useful for ensuring penetration of a subject, whilst providing frequencies that attenuate in human tissue without significant damage/injury to the tissue. The reference electrode serves as an electric reference for all measurements taken by electrodes, e.g. as a reference for the response of the internal electrodes.

The electrical response of an internal electrode to the electric fields changes as the relative position of the internal electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the catheter using a mapping system 110 that monitors a response of any internal electrodes to the crossing electric fields, e.g. by mapping the (electrical) response of an electrode to a relative position within the subject or using a suitable mapping/transfer function, e.g. the "V2R function", to determine the relative position(s) of the electrodes, and therefore the catheter, in a defined space. This defined space is sometimes called an R-space.

Approaches for generating such a mapping function are well established in the art. Example processes are disclosed by the European Patent Applications EP 0775466 A2, EP 3568068 A1 and EP 3607879 A1.

Once the relative locations of the catheter and/or one or more sensors/electrodes on the catheter have been established, it is possible to construct an anatomical model 115 of an anatomical cavity (i.e. a cavity in which the interventional device is able to move). This process is also performed by the mapping system 110.

Broadly, the mapping system 110 may build an anatomical model of an anatomical cavity 101 (e.g. a chamber, vessel or void) within a subject by processing a cloud of points. Each point represents a position of the catheter or sensor/electrode in a defined space. More specifically, a reconstruction algorithm is used to generate an anatomical model of the (investigated part of the) anatomical cavity. The anatomical model may, for example, be a 3D surface that depicts or models the (known bounds of) the anatomical cavity.

For the avoidance of doubt, it is noted that an anatomical model is a dataset containing information that defines a 3D surface (e.g. within a predefined co-ordinate system), such as a 3D mesh or the like. The anatomical model therefore represents data (representing a 3D surface) that can be processed by a data processor or the like.

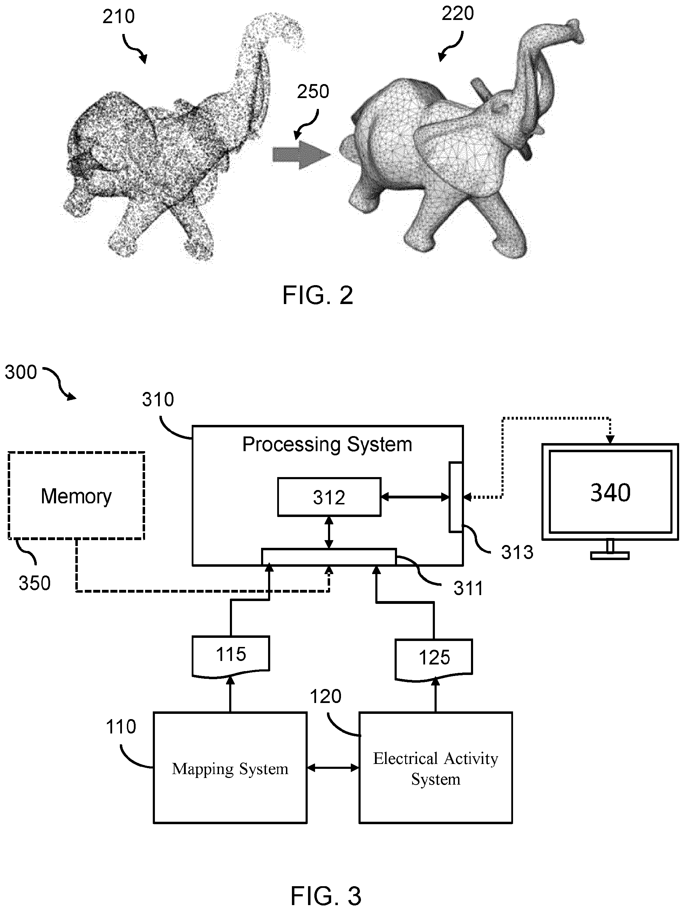

The process of reconstructing an anatomical model from a point cloud is conceptually illustrated in Figure 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

Any model data representing an anatomical model can thus comprise the raw measurement data obtained by any catheter having position sensors. In case of the dielectric imaging or mapping technique such measurement data typically comprise electrical measurements representative of the electric field applied to the anatomical element during roving around of the catheter within the cavity of the anatomical element. Such electrical data may then be transformed into positions of the position sensors (in this case catheter electrodes) in space according to known methods as described and referred to herein. From the positional data the model may be reconstructed as described using known methods as referred to and described herein. Similar to the dielectric imaging technique, in case of magnetic localization technique the magnetic measurement data recorded by the catheter positional sensors in the magnetic field applied to the anatomical element may be used to generate a positional point cloud from which the anatomical model may be reconstructed. The processing systems as described herein are typically also configured to perform such steps from processing model data into anatomical models.

Turning back to Figure 1, the electrical activity system 120 is configured to generate a measured electrical activity model 125. This is performed by using one or more electrical sensors 151, 152, 153 (e.g. electrodes) mounted in/on the catheter 155 to measure electrical activity of the anatomical element as the catheter moves within the anatomical cavity.

A measurement of electrical activity is a measurement of any electrophysical characteristic at a location of the anatomical element. A measurement of electrical activity may include a voltage, current or impedance measurement. Any other suitable measure of electrical activity would be apparent to the skilled person, and may be specific to the type of anatomical element.

For instance, where the anatomical element is a heart, the measure of electrical activity may be a (relative) activation time at a particular location. An activation time represents the time at which an activation wave passes that location, i.e. a difference between a time at which a (natural or artificial) cardiac pacemaker activates (i.e. the heart pulses or is paced) and a time at which that location activates or shows an electrical change.

As another example, the measure of electrical activity could be an amplitude or pulse shape of electrical activity, e.g. a measure of peak width, an identifier of peak shape (e.g. sawtooth, sinusoidal etc.) and so on.

If crossing electric fields are used to determine a location of the catheter or electrodes on the catheter, it will be appreciated that the electrical response to the electric fields is in a different frequency range to the electrical response to the electrical activity of the anatomical element. This allows the systems 110, 120 to distinguish between different types of electrical response, namely responses to induced electrical fields within the anatomical element and responses to electrical activity of the anatomical element itself.

The measured electrical activity model may be generated by using the anatomical model as a base structure, and overlaying measured electrical activities at different points of the anatomical model to generate the measured electrical activity model. In particular, each time a measure of electrical activity is taken, the location of the measurement (with respect to the model) can be determined and used to provide a location for the measure in the measured electrical activity model. In some approaches, interpolation is performed to predict the values for the electrical activity model at positions lying between positions for which a measure of the electrical activity model was obtained/sampled.

Thus, the electrical activity model is a dataset containing information that defines values for one or more parameters of electrical activity with respect to a 3D surface (e.g. within a predefined co-ordinate system) that represents the bounds of the anatomical structure such as a 3D mesh or the like. The electrical activity model is therefore data that can be processed by a data processor or the like.

Other approaches for generating an electrical activity model using measures of electrical activity from a catheter would be readily apparent to the person skilled in the art. Indeed, approaches for performing feature extraction and interpolation would be known to the person skilled in the art.

It will be appreciated that the mapping system 110 and the electrical activity system 120 may form aspects of a single processing system, e.g. represents different processes performed by a single processing system.

Figure 3 illustrates a system 300 according to an embodiment of the disclosure.

The system comprises a processing system 310 that processes the anatomical model 115 and the measured electrical activity model 125 produced by the mapping system 110 and the electrical activity system 120 respectively. The processing system 310 may be in the form of a workstation.

It will be appreciated that the mapping system 110, the electrical activity system 120 and/or the processing system 310 may form aspects of a single system, e.g. each form different subsystems or processes performed by the single system. In the context of the present disclosure, this means that a single system could perform the actions of a mapping system, an electrical activity system and/or a processing system as herein described. Such systems known in the art comprise the CARTO^{®} system of Biosense Webster^{®}, the EnSite^{®} system of Abbott^{®} and the KODEX-EPD^{®} system of Philips^{®}, but others exist. Although not shown in Figure 3, in such cases the processing system may also be configured to control any field generating systems such as the electric field generator 139 and/or field generating system 130.

The processing system 310 comprises an input interface 311 that receives the two models 115, 125. These models may be received directly from the mapping system 110 and the electrical activity system 120, or may be received from a memory or storage unit 350. The memory or storage unit may, in its turn, receive the models from the mapping system 110 and the electrical activity system 120.

The processing system 310 also comprises a processing unit 312 that is communicatively coupled to the input interface.

The processing unit 312 is configured to process the anatomical model 115 using an electrophysical simulation technique to generate a predicted electrical activity model.

In other words, the processing unit 312 uses the anatomical model 115 to simulate or model electrophysical activity of the anatomical element. Any suitable electrophysical simulation technique can be employed to carry out this process. More particularly, it has been shown that finite-element computations can be used to predict the electrical activity over the heart. This electrophysiological simulation can be performed using the anatomical model as an input, e.g. as an input mesh.

The electrophysical simulation technique may comprise a simulation of electrical activity in an anatomical structure defined by the anatomical model. Thus, the anatomical model may effectively be used as a representation of the anatomical structure.

In some examples, the electrophysical simulation technique comprises using values contained in the measured electrical activity model as boundary conditions for the simulation of electrical activity. In other words, electrophysical simulation technique may also process the measured electrical activity model to generate the predicted electrical activity model. Preferably, if this approach is used, not all of the values of the measured electrical activity model are used, for reasons explained below.

In one example, where the anatomical element is a heart and the measures of electrical activity are measures of local activation times, the electrophysical simulation technique may comprise a process of simulating local point-to-point propagation of an electrical impulse with respect to a heart structure represented by the anatomical model to generate predictive relative activation times between different areas of the heart structure represented by the anatomical model. Correspondingly, the measured electrical activity model may comprise measures of relative activation times between the different areas of the heart structure represented by the anatomical model.

Other example approaches for simulating electrical activity using an anatomical model are established in the art. Approaches may adopt any mechanism disclosed by, inter alia:
B Baillargeon, N Rebelo, D Fox, R Taylor, E Kuhl (2014) The Living Heart Project: A robust and integrative simulator for human heart function, European Journal of Mechanics - A/Solids. 48:38-47; Kotikanyadanam, M., Göktepe, S., Kuhl, E., 2010. Computational modeling of electrocardiograms: a finite element approach towards cardiac excitation. Int. J. Num. Meth. Biomed. Eng. 26, 524e533; Cansiz, B., Sveric, K., Ibrahim, K., Strasser, R. H., Linke, A., & Kaliske, M. (2018). Towards predictive computer simulations in cardiology: Finite element analysis of personalized heart models. ZAMM-Journal of Applied Mathematics and Mechanics/Zeitschrift für Angewandte Mathematik und Mechanik, 98(12), 2155-2176; Arevalo HJ, Vadakkumpadan F, Guallar E, Jebb A, Malamas P, Wu KC, Trayanova NA. (2016) Arrhythmia risk stratification of patients after myocardial infarction using personalized heart models. Nat Commun. 7:11437 and so on.

These examples provide a small selection of suitable simulation techniques that make use of an anatomical model to simulate electrical activity. The skilled person would be readily capable of selecting one or more other approaches to analyzing models to generate simulated or predicted values of electrical activity.

The processing unit 312 is configured to identify any regions of mismatch between the predicted electrical activity model and the measured electrical activity model. The regions of mismatch represent regions of the anatomical element that are either insufficiently represented by the anatomical model and/or sites of a local structural or electrical abnormality in the anatomical element.

It is recognized that the simulated/predicted electrical activity and the measured electrical activity should be near identical (within a reasonable margin of error). Differences between the two must thereby indicate areas, zones or regions where there is an abnormality (e.g. due to an unexpected or unusual structure or electrical response of the anatomical element) or where insufficient mapping has been performed - such that the anatomical model is inaccurate.

To account for minor errors that are to be expected due to noise or intrinsic inaccuracies in prediction mechanisms, a region of mismatch may be defined as a region in which a difference between a value of the predicted electrical activity model and the measured electrical activity model differs by more than a predetermined amount. The predetermined amount may, for instance, be a percentage of the largest or smallest of the two values (e.g. ± 5% or ±10%). Other suitable predetermined amounts would be apparent to the skilled person.

Determining the location or existence of any such regions of mismatch thereby provides valuable information for a clinician, to guide them to areas that require additional attention and/or mapping. This thereby provides valuable information for treating and/or assessing the subject.

It has previously been mentioned how, if the measured electrical activity model is used in the generation of the predicted electrical activity model, then it is preferable for not all of the values of the measured electrical activity model to be used. This is because this allows the unused parts of the measured electrical activity model to be used in identifying areas of mismatch (as otherwise, the predicted electrical activity model might be identical to the measured electrical activity model).

In this way, some of the values of the measured electrical activity model may be used as boundary conditions in the generation of the predicted electrical activity model, with other values being used to assess or identify regions of mismatch (i.e. regions of mismatch).

In some examples, the processing unit 312 may be further configured to generate an uncertainty map identifying the position of the regions of mismatch with respect to the anatomical model. The relative location of each region of mismatch with respect to the anatomical model can be readily determined, particularly if the measured activity model is/was generated using the anatomical model. Such an uncertainty map would provide a clinician with useful information for easily and readily identifying regions that would benefit from further investigation and/or analysis. In particular, an uncertainty map may identify areas worthy of further mapping (e.g. to update and/or correct the anatomical model).

To improve the accuracy of the measured electrical activity model (and therefore a true identification of potential abnormalities), arrhythmic cardiac activity may be identified and used to filter the measurements used in the measured electrical activity model. This would reduce any inaccurate identifications of potential regions of abnormalities.

Accordingly, in some examples, the input interface 311 is further configured to receive electrocardiogram data or other electrical response data identifying any occurrences of arrhythmic cardiac activity. The processing unit 312 may be similarly configured to discount or remove, from the measured electrical activity model, any measures of electrical activity measured during any identified occurrence of arrhythmic cardiac activity.

Optionally, the processing unit 312 may be configured to generate, for each identified region of mismatch, an indicator of the severity of the mismatch. This indicator of severity may be based on (e.g. equal to) the difference between the predicted electrical activity, provided by the predicted electrical activity model, and the measured electrical activity, provided by the measured electrical activity model, at the region of mismatch.

A measure of severity provides valuable information for a clinician or further processing system to prioritize and/or assess an importance of a region of mismatch. In particular, a measure of severity may indicate a likelihood that a particular region exhibits an abnormality or that mapping in that location has not been performed accurately or completely (i.e. that the anatomical model is inaccurate in that area).

In some examples, the processing unit 312 may be configured to generate, for each identified region of mismatch, a predictive indicator of whether the identified region has been insufficiently sampled or whether the identified region is a site of a local structural or electrical abnormality in the anatomical element.

The predictive indicator may be generated by using additional information about the identified region.

For instance, a measure of impedance of the identified region may be used to determine whether the identified region exhibits fibrosis. A high level of impedance (i.e. impedance exceeding some threshold that may be defined relative to other parts of the anatomical element) indicates the presence of fibrosis. This would indicate that the identified region of mismatch is more likely to represent a site of a local structural or electrical abnormality in the anatomical element than an unsampled region. In this way, a predictive indicator may use additional information, such as impedance measurements, to discriminate between undersampled regions and structural/electrically abnormal regions.

The measure of impedance may be obtained during determination of the electrical response of one or more electrodes of the catheter (i.e. when the locations of the catheter are being obtained).

In some examples, the processing system 310 further comprises an output interface 313 configured to provide output information responsive to the identified regions of mismatch. The output information may contain, for instance, information identifying the identified regions or interest and/or further information derived by processing this information.

The output interface 313 may be configured to control a user interface 340 that provides a user-perceptible output (e.g. a sound and/or visual display) responsive to the output information. However, this is not essential.

In other examples, the output interface may provide the output information to a further processing system. It is recognized that the identified regions of mismatch may provide valuable information for other automated systems, e.g. automated diagnosis systems, such that it may be advantageous to simply provide the output information to a further processing system.

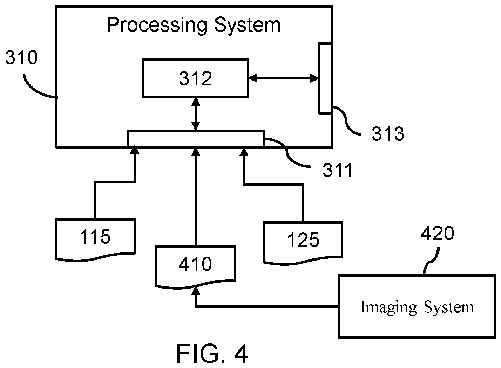

Figure 4 illustrates an optional variation for the processing system 310.

In this variation, the input interface 311 is further configured to receive a pre-operative anatomical model 410. The pre-operative anatomical model 410 is/was generated from a non-invasive imaging procedure, e.g. performed by an imaging system 420.

The processing unit 312 may be further configured to spatially register the pre-operative anatomical model to the anatomical model; and identify any regions of the anatomical element represented in the pre-operative anatomical model but not represented in the anatomical model. This facilitates identification of regions of an anatomical structure containing the anatomical element that have not yet been explored.

One approach for spatially registering the pre-operative anatomical model to the anatomical model is to identify the same one or more landmark features in the pre-operative anatomical model and the anatomical model; and based on the identified landmark features, spatially registering the pre-operative anatomical to the anatomical model. Another approach uses a machine-learning method to spatially register the two models together.

In some examples, the pre-operative anatomical model is processed using an electrophysical simulation technique to generate a second predicted electrical activity model. The second predicted activity model may be compared to the predicted electrical activity model and/or the measured electrical activity model to identify further regions of mismatch.

This embodiment appreciates that the pre-operative anatomical model may represent a larger extent of the anatomical element, and may be more accurate in representing the overall anatomical element. Thus, any regions of mismatch between the second predicted electrical activity model and the predicted electrical activity model are likely to represent areas that are insufficiently represented by the anatomical model. Similarly, any regions of mismatch between the second predicted electrical activity model and the measured electrical activity model are likely to represent sites of a local structural or electrical abnormality in the anatomical element.

This approach thereby provides further and improved identification of potential regions of the anatomical element that are either insufficiently represented by the anatomical model and/or sites of a local structural or electrical abnormality in the anatomical element.

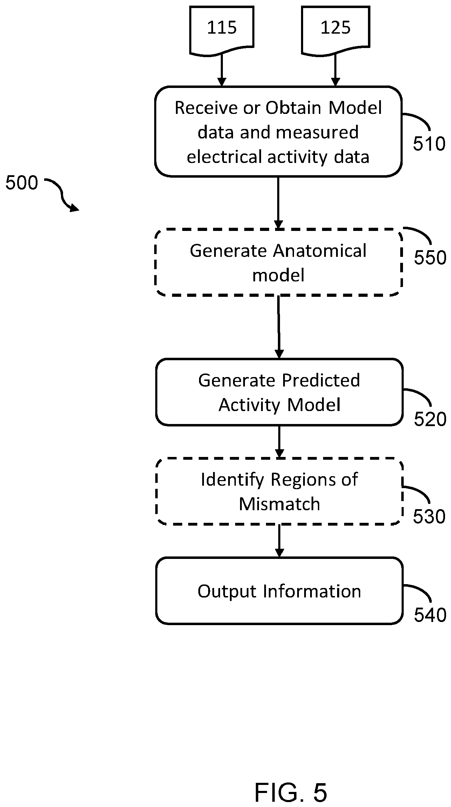

Figure 5 illustrates methods 500 according to embodiments of the disclosure. The methods 500 may be executed by any herein described processing system and be implemented in hardware, computer code (software) or a combination thereof.

The method 500 comprises a step 510 of receiving, at an input interface: an anatomical model 115 in the form of model data representative of the anatomical model of the anatomical element and measured electrical activity data for example including measured electrical activity model 125. The anatomical model is/was generated by monitoring the location of a catheter as the catheter moves within an anatomical cavity of the anatomical element. The measured electrical activity model is/was generated by using an electrical sensor mounted in or on the catheter to obtain measures of electrical activity of the anatomical element as the catheter moves within the anatomical cavity of the anatomical element.

In a basic form, the model data comprise the anatomical model without substantial data processing, i.e. it defines the anatomical model directly. However, in other embodiments the anatomical model is generated from the model data. In such case there is the optional step 550. Such generation may comprise processing measurement data of a mapping system as described herein before to provide a data set of positions of position sensors in space and subsequent generation of the anatomical model from such data set.

The input interface may receive the models 115, 125 from a memory or storage unit and/or directly from systems that generate the models from information obtained from the catheter.

The method 500 further comprises a step 520 of processing, using a processing unit, the anatomical model using an electrophysical simulation technique to generate a predicted electrical activity data or model. Approaches for performing step 520 have been previously described.

The method further comprises an optional, but preferred step 530 of identifying, using the processing unit, any regions of mismatch between the predicted electrical activity model and the measured electrical activity model, to thereby identify any regions of the anatomical element that are either insufficiently represented by the anatomical model and/or sites of a local structural or electrical abnormality in the anatomical element.

The method 500 may further comprise a step 540 of optionally providing, at an output interface, output information responsive to the identified regions of mismatch.

Method 500 may be iteratively repeated, e.g. by the processing system, as the models are updated, e.g. as further investigations of the anatomical element are performed using the catheter. However, this is not essential, and method 500 may instead be performed independently of the operation of the catheter.

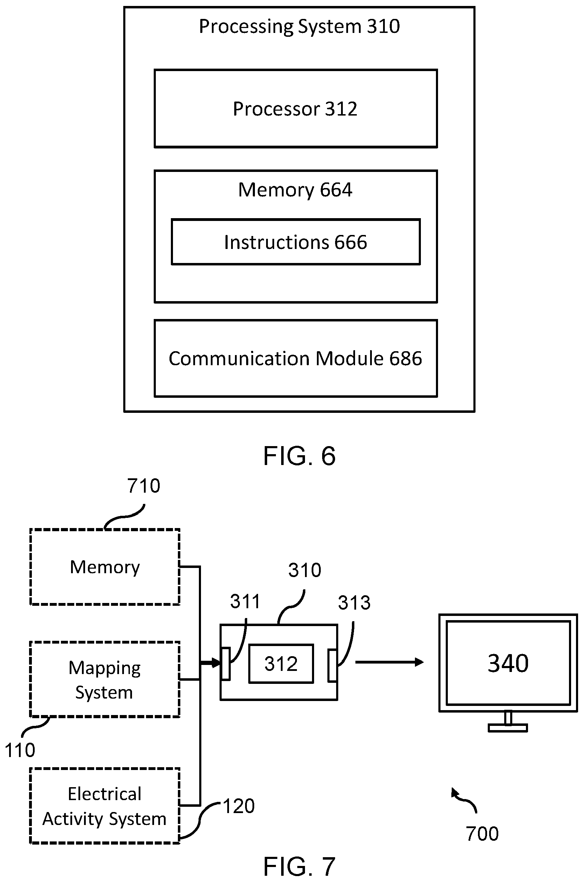

Figure 6 is a schematic diagram of a processing system 310, according to embodiments of the present disclosure. The processing system 310 is configured to carry out a method according to an embodiment, such as the method 500 described with reference to Figure 5.

As shown, the processing system 310 may include a (data) processing unit 312, a memory 664, and a communication module 668. These elements may be in direct or indirect communication with each other, for example via one or more buses.

The processing unit 312 may include a central processing unit (CPU), a digital signal processor (DSP), an ASIC, a controller, an FPGA, another hardware device, a firmware device, or any combination thereof configured to perform the operations described herein. The processing unit 312 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 664 may include a cache memory (e.g., a cache memory of the processing unit 312), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 664 includes a non-transitory computer-readable medium. The non-transitory computer-readable medium may store instructions. For example, the memory 664, or non-transitory computer-readable medium may have program code recorded thereon, the program code including instructions for causing the processing system 310, or one or more components of the processing system 310, particularly the processing unit 312, to perform the operations described herein. For example, the processing system 310 can execute operations of the method 500.

Instructions 666 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 664, with the code recorded thereon, may be referred to as a computer program product.

The communication module 686 can include any electronic circuitry and/or logic circuitry to facilitate direct or indirect communication of data between the processing system 310, the penetration device and/or the user interface (or other further device). In that regard, the communication module 668 can be an input/output (I/O) device. In some instances, the communication module 668 facilitates direct or indirect communication between various elements of the processing system 310 and/or the system (Figure 7).

Figure 7 illustrates a system 700 according to an embodiment of the disclosure, which illustrates some optional elements. The system 700 comprises a processing system 310, which may be embodied as previously described.

The system may further comprise an output display 340. The processing system may be configured to control the output display 340 to display a visual representation of any data generated by the processing system 310, such as any identified regions of mismatch.

This control may be performed using an output interface 313 of the processing system 310. The visual representation of the determined position(s) may be positioned with respect to the anatomical model, e.g. to indicate a relative position of the within the anatomical cavity represented by the anatomical model.

The processing system 310 may be adapted to obtain the anatomical model and/or the measured electrical activity model from a memory 710 and/or systems 110, 120 that generate such models, e.g. at an input interface 311. The system 700 may comprise the memory and/or the systems 110, 120 as appropriate.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored there on the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

It will be appreciated that different disclosed systems may form part of a single processing system, e.g. each disclosed system is a sub-system of a single processing system. In the context of the present disclosure, this means that a single system could perform the actions of a mapping system, an electrical activity system and/or a processing system as herein described. An input/output interface may represent internal circuitry of a larger system, e.g. connected to an input/output of a processing unit. The same processing unit may be reused to perform different tasks, such as: generating the anatomical model, generating the electrical activity model, generating a predicted electrical activity model and/or identifying regions of mismatch.

Variations to the disclosed embodiments can be understood and effected by those skilled in the art in practicing the claimed disclosure, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. A processing system (310) for aiding an analysis of an anatomical element (101) of a subject (105), the processing system comprising:
- an input interface (311) configured to receive (510):
- model data representative of an anatomical model of at least a part of the anatomical element; and
- electrical activity data representative of a measured electrical activity of the at least a part of the anatomical element;
- a processing unit (312) communicatively coupled to the input interface and configured to:
- process (520) the model data using an electrophysical simulation technique to generate predicted electrical activity data for the at least a part of the anatomical element;
- optionally, an output interface (313) configured to provide (540) output data representative of a comparison of the at least some of the predicted electrical activity data with at least some of the corresponding measured electrical activity data.

2. The processing system of claim 1, wherein:
- the model data comprises the anatomical model; or
- the processing unit is configured to process the model data to generate the anatomical model; and
- the processing unit is configured to process at least the anatomical model using the electrophysical technique to generate the predicted electrical activity data.

3. The processing system of claim 1 or 2, wherein the processing unit is configured to, based on the comparison of the at least some of the predicted electrical activity data and the measured electrical activity data, identify one or more regions of mismatch between the at least some of the predicted electrical activity data and the measured electrical activity data, and optionally, wherein the output data is indicative of the one or more regions of mismatch.

4. The processing system of any one of claim 1 to 3, wherein a region of mismatch comprises or is a region in which a difference between a value of the predicted electrical activity data and the measured electrical activity data differs by more than a predetermined amount.

5. The processing system of any one of the previous claims, wherein the electrophysical simulation technique comprises a finite-element simulation that models electro-activation to generate the predicted electrical activity data.

6. The processing system of any one of the previous claims, wherein the processing unit is further configured to generate an uncertainty map identifying the position of one or more of the one or more regions of mismatch with respect to the anatomical model.

7. The processing system of any of the previous claims, wherein the electrophysical simulation technique comprises using values contained in the measured electrical activity data as boundary conditions for the simulation of electrical activity data.

8. The processing system of any of claims 1 to 7, wherein:
- the input interface is further configured to receive pre-operative imaging data representative of a pre-operative anatomical model of the anatomical element, the pre-operative imaging data being the result of a non-dielectric imaging procedure and/or of a non-invasive imaging procedure; and
- the processing unit is further configured to:
- spatially register the pre-operative anatomical model to the anatomical model; and
- compare identify any regions of the anatomical element represented in the pre-operative anatomical model but not represented in the anatomical model.

9. The processing system of any of claims 1 to 8, wherein:
- the input interface is further configured to receive pre-operative anatomical data (410) resulting from an imaging procedure of the anatomical element; and
- the processing unit is further configured to:
- process the pre-operative anatomical data using an electrophysical simulation technique to generate second predicted electrical activity data for the at least part of the anatomical element;
- identify any regions of mismatch between the second predicted electrical activity data and the measured electrical activity data.

10. The processing system of any of claims 1 to 9, wherein:
the input interface is further configured to receive electrocardiogram data identifying any occurrences of arrhythmic cardiac activity; and
the processing unit is configured to discount, from the measured electrical activity model, any measures of electrical activity measured during any identified occurrence of arrhythmic cardiac activity.

11. The processing system of any of claims 1 to 10, wherein the processing unit is configured to generate, for each identified region of mismatch, an indicator of the severity of the mismatch based on the difference between the predicted electrical activity, provided by the predicted electrical activity model, and the measured electrical activity, provided by the measured electrical activity model, at the region of mismatch.

12. The processing system of any of the previous claims further comprising a user interface configured to be communicative coupled to the output interface and to provide the at least part of the anatomical model and the comparison to a user.

13. A medical treatment system comprising the processing system of any of the previous claims.

14. A computer-implemented method (500) for aiding an analysis of an anatomical element of a subject, the computer-implemented method comprising:
- receiving (510), at an input interface (311):
- model data representative of an anatomical model of at least a part of the anatomical element; and
- electrical activity data representative of a measured electrical activity of the at least part of the anatomical element;
- processing (520), using a processing unit (312) communicatively coupled to the input interface:
the model data using an electrophysical simulation technique to generate predicted electrical activity data for at least part of the anatomical element;
- optionally, providing (540) at an output interface (313) output data representative of a comparison of the at least some of the predicted electrical activity data with at least some of the corresponding measured electrical activity data.

15. A computer program product comprising computer readable program code means which, when executed by a processing unit of a processing system, cause the processing system to perform all of the steps of the method of claim 14.
